(19)

**Europäisches Patentamt**
**European Patent Office**
**Office européen des brevets**

(11) **EP 4 453 945 B1**

(12) **EUROPEAN PATENT SPECIFICATION**

(45) Date of publication and mention
of the grant of the patent:
**26.11.2025 Bulletin 2025/48**

(21) Application number: **22844086.3**

(22) Date of filing: **22.12.2022**

(51) International Patent Classification (IPC):
**G16B 40/30** *(2019.01)* **G16B 40/10** *(2019.01)*
**G16B 25/10** *(2019.01)* **G16B 20/00** *(2019.01)*

(52) Cooperative Patent Classification (CPC):
**G16B 25/10; G16B 20/00; G16B 40/10;**
**G16B 40/30**

(86) International application number:
**PCT/EP2022/087555**

(87) International publication number:
**WO 2023/118473 (29.06.2023 Gazette 2023/26)**

(54) **METHODS FOR CLUSTERING MELTING CURVES TO IDENTIFY GENOTYPES**

VERFAHREN ZUM CLUSTERN VON SCHMELZKURVEN ZUR IDENTIFIZIERUNG VON
GENOTYPEN

PROCÉDÉS POUR REGROUPER DES COURBES DE FUSION POUR IDENTIFIER DES
GÉNOTYPES

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **22.12.2021 US 202163265848 P**

(43) Date of publication of application:
**30.10.2024 Bulletin 2024/44**

(73) Proprietors:
• **F. Hoffmann-La Roche AG**
  **4070 Basel (CH)**
  Designated Contracting States:
  **AL AT BE BG CH CY CZ DK EE ES FI FR GB GR**
  **HR HU IE IS IT LI LT LU LV MC ME MK MT NL NO**
  **PL PT RO RS SE SI SK SM TR**
• **Roche Diagnostics GmbH**
  **68305 Mannheim (DE)**
  Designated Contracting States:
  **DE**

(72) Inventor: **KURNIK, Ronald**
**Pleasanton, California 94588 (US)**

(74) Representative: **Mewburn Ellis LLP**
**Aurora Building**
**Counterslip**
**Bristol BS1 6BX (GB)**

(56) References cited:
**WO-A1-2005/071113    WO-A2-2004/038038**
**WO-A2-2007/035806**

• **MICHAEL LIEW ET AL: "Genotyping of Single-Nucleotide Polymorphisms by High-Resolution Melting of Small Amplicons", CLINICAL CHEMISTRY, OXFORD UNIVERSITY PRESS, US, vol. 50, no. 7, 1 January 2004 (2004-01-01), pages 1156 - 1164, XP008129908, ISSN: 0009-9147, DOI: 10.1373/CLINCHEM.2004.032136**

**Description**

**TECHNICAL FIELD**

The present disclosure relates to methods for genotyping melting curves, particularly to methods for automatically clustering melting curves to improve genotyping of the melting curves.

**BACKGROUND**

The polymerase chain reaction (PCR) has become a ubiquitous tool of biomedical research, disease monitoring and diagnostics. Melting curve analysis has similarly become a common tool used to identify DNA genotypes, often performed after PCR. Melting curve analysis assesses the dissociation characteristics of double strand DNA during heating. In particular, fluorescent dyes bound to double strand DNA typically lose fluorescence as the temperature increases and exhibit a reduction in fluorescence that coincides with the effective dissociation of the DNA. Since different genotypes dissociate at different temperatures, different genotypes thus have melting curves with different profiles. The temperature at which this effective dissociation occurs is often ascertained by identifying peaks formed in the negative first derivative of the melting curve. Thus, negative first derivatives that have similar features (such as the peaks) may indicate curves belonging to the same genotype. Accordingly, analysis of the melting curves (and particularly the negative first derivative thereof) can be used to genotype the assay by grouping together similar curves.

While methods exist for such genotyping based on melting curve analysis, the previous methods have limitations. For example, existing methods are of limited effectiveness when dealing with a diversity of genotypes, including assays with a large number of genotypes and/or instances where the quality of the melting curves is inconsistent. In this context, patent publication WO 2007/035806 A2 discloses an automated method for genotyping a plurality of melting curves comprising fluorescent measurements over a range of temperatures, the method comprising generating a plurality of processed melting curves by calculating the negative first derivative of each of the plurality of melting curves over the range of temperatures and clustering said melting curves based on a distance metric in order to identify genotypes. Accordingly, there is a need for an automated method for genotyping melting curves in assays with large numbers of genotypes and/or where the quality of the melting curves is inconsistent.

**SUMMARY**

The present disclosure provides for novel methods for clustering melting curves to improve melting curve genotyping. The methods are performed by processing the melting curves, compiling difference matrices by comparing the curves, compiling cluster matrices based on the difference matrices, filtering the cluster matrices, and identifying genotypes based on the filtered cluster matrices.

In an aspect, an automated method for genotyping a plurality of melting curves is provided. The melting curves may include fluorescent measurements over a range of temperatures. The method includes generating a plurality of processed melting curves by calculating the negative first derivative of each of the plurality of melting curves over the range of temperatures. The method further includes compiling a first difference matrix based on the processed melting curves by calculating, for each of the processed melting curves, a sum of absolute differences between a given processed melting curve and each of the other processed melting curves over the range of temperatures. The method further includes compiling a second difference matrix based on the first difference matrix by summing, for each column of the first difference matrix, each row vector over the range of temperatures. The method further includes compiling an initial cluster matrix based on the second difference matrix, wherein each row in the initial cluster matrix identifies an initial cluster in the set of initial clusters. The method includes filtering the initial cluster matrix into a filtered initial cluster matrix comprising a set of filtered initial clusters, wherein each of the processed melting curves is assigned to a single filtered initial cluster. The method further includes calculating a number of peaks and a mean melt temperature of each peak in each filtered initial cluster of the filtered initial cluster matrix. The method further includes combining filtered initial clusters with the same number of peaks and a mean melt temperature within a specified temperature threshold to compile a final cluster matrix and identifying a genotype of each of the plurality of melting curves based on the final cluster matrix.

In some embodiments, the initial cluster matrix is compiled by performing k-means clustering. For example, each row of the initial cluster matrix may include a first column identifying a first processed melting curve, a second column identifying a second processed melting curve with a minimum non-zero distance from the first processed melting curve of the first column, and subsequent columns identifying additional processed melting curves within a cluster with the first processed melting curve of the first column. The minimum non-zero distance may be determined based on the second difference matrix, and the cluster may be identified based on the distance between a centroid of the cluster and the first processed melting curve.

In some embodiments, filtering the initial cluster matrix includes removing duplicate rows from the initial cluster matrix. In some embodiments, filtering the initial cluster matrix further includes combining intersecting clusters that contain common processed melting curves. For example, combining intersecting clusters may include the steps of (a) identifying, in a first pass, initial clusters in the set of initial clusters that contain a common processed melting curve, (b) combining each of the clusters identified in step (a), (c) ) identifying, in a second pass, any remaining clusters that contain common processed melting curves and combining said remaining clusters, and (d) removing the initial clusters containing common processed melting curves such that none of the processed melting curves are present in more than one filtered initial cluster. In some embodiments, filtering the initial cluster matrix includes identifying clusters with duplicate processed melting curves, and removing said duplicates.

In some embodiments, filtering the initial cluster matrix includes the steps of: (i) identifying a negative cluster based on a comparison of a maximum signal of the cluster to a cluster threshold, and (ii) merging any negative clusters identified in step (i). For example, identifying a negative cluster may include determining a ratio of a maximum fluorescence value in a given cluster and a maximum fluorescence in a cluster with the highest fluorescence and comparing the ratio determined at step (aa) to the cluster threshold. For example, the cluster threshold may be 0.1.

In some embodiments, filtering the initial cluster matrix may further include identifying a cluster comprising a peak that is less than the peaks of each of the other clusters, comparing processed melting curves in the cluster identified in step (iii) to the cluster threshold, and setting any of the processed melting curves that are less than the cluster threshold as negative processed melting curves and removing said negative processed melting curves from the cluster.

In some embodiments, the specified temperature threshold may be 1 ° C.

In some embodiments, quality metrics may be determined. For example, the method may further include determining a quality metric associated with each of the identified genotypes according to the formula:

$$qMetric = round\left\{100\left[1-\frac{stdev\left(grp\_curves\right)}{mean\left(grp\_curves\right)}\right]\right\}$$

wherein "grp_curves" comprises a group of melting curves within a given cluster. As another example, the method may further include determining a quality metric associated with each processed melting curve within a cluster according to the formula: $$qMetric = 1-0.25\left[\frac{abs\left(mean\left(f-median(f)\right)\right)}{mean\left(stdev(f)\right)}\right]$$ wherein "f" designates a given processed melting curve.

In some embodiments, the initial cluster matrix is compiled using clustering techniques. For example, the initial cluster matrix may be compiled by performing at least one of hierarchical clustering, fuzzy C means clustering, mean shift clustering, density-based spatial clustering, and Gaussian mixture models.

In another aspect, a system for automated genotyping of melting curves is provided. The system may include at least one data processor. The system may further include at least one memory story instructions, which when executed by the at least one data processor, cause operations including generating a plurality of processed melting curves by calculating the negative first derivative of each of the plurality of melting curves over the range of temperatures, compiling a first difference matrix based on the processed melting curves by calculating, for each of the processed melting curves, a sum of absolute differences between a given processed melting curve and each of the other processed melting curves over the range of temperatures. The operations may further include compiling a second difference matrix based on the first difference matrix by summing, for each column of the first difference matrix, each row vector over the range of temperatures. The operations may further include compiling an initial cluster matrix based on the second difference matrix, wherein each row in the initial cluster matrix identifies an initial cluster in the set of initial clusters. The operations may further include filtering the initial cluster matrix into a filtered initial cluster matrix comprising a set of filtered initial clusters, wherein each of the processed melting curves is assigned to a single filtered initial cluster. The operations may further include calculating a number of peaks and a mean melt temperature of each peak in each filtered initial cluster of the filtered initial cluster matrix. The operations may further include combining filtered initial clusters with the same number of peaks and a mean melt temperature within a specified temperature threshold to compile a final cluster matrix and identifying a genotype of each of the plurality of melting curves based on the final cluster matrix.

**BRIEF DESCRIPTION OF THE DRAWINGS**

FIG. 1 is a graphical representation of raw melting curves to be genotyped in accordance with embodiments of the present disclosure.

FIG. 2 is a graphical representation of processed raw melting curves of FIG. 1 to be genotyped in accordance with embodiments of the present disclosure.

FIG. 3 is a graphical representation of processed raw melting curves of FIG. 2 after being genotyped in accordance with embodiments of the present disclosure.

FIG. 4 shows a method for automated genotyping of melting curves in accordance with embodiments of the present disclosure.

FIG. 5 is a graphical representation of the compilation of a difference matrix used in the method of FIG. 4 in accordance with embodiments of the present disclosure.

FIG. 6 is a graphical representation of the compilation of a difference matrix used in the method of FIG. 4 in accordance with embodiments of the present disclosure.

FIG. 7 is a graphical representation of the compilation of a cluster matrix used in the method of FIG. 4 in accordance with embodiments of the present disclosure.

FIG. 8 is a graphical representation of genotyped processed raw melting curves in accordance with embodiments of the present disclosure.

FIG. 9 is a graphical representation of genotyped processed raw melting curves in accordance with embodiments of the present disclosure.

FIG. 10 is a graphical representation of genotyped processed raw melting curves in accordance with embodiments of the present disclosure.

FIG. 11 is a graphical representation of genotyped processed raw melting curves in accordance with embodiments of the present disclosure.

FIG. 12 is a graphical representation of genotyped processed raw melting curves in accordance with embodiments of the present disclosure.

FIG. 13 is a diagram illustrating an example of a genotyping platform in accordance with embodiments of the present disclosure.

FIG. 14 is a block diagram of a computing system in accordance with embodiments of the present disclosure.

## DETAILED DESCRIPTION

In the context of in vitro diagnostic (IVD) assays, it is desirable to determine different genotypes. To that end, and as described above, post-PCR melting steps have been used to determine different genotypes of a given assay. Since the fluorescent dyes bound to double strand DNA lose fluorescence as the temperature increases depending on the characteristics thereof, different genotypes of a given assay will exhibit different fluorescent signal profiles. In order to use melting curves to determine genotypes, typically, the raw melting curve, *i.e.,* the measured fluorescence over the range of temperatures during the melting step, is processed by taking the negative first derivative thereof. This results in curves with a series of peaks. The peaks are usually Gaussian shaped peaks, but can be peaks of other forms, such as Lorentzian, Voigt, Pearson, Compton Edge, or any other types of peaks. Curves which have peaks that resemble each other in shape and frequency, and curves with similar melting temperatures can be grouped together in a single genotype. While existing methods may be able to identify genotypes from a limited number of genotypes, there is still a desire for an automated method for determining genotypes when there is an unknown and/or relatively large number of genotypes for a given assay. For example, a given assay may have anywhere from one to twelve different genotypes, and existing methods are not sufficient to automatically determine genotypes from the melting curves where the genotypes are unknown.

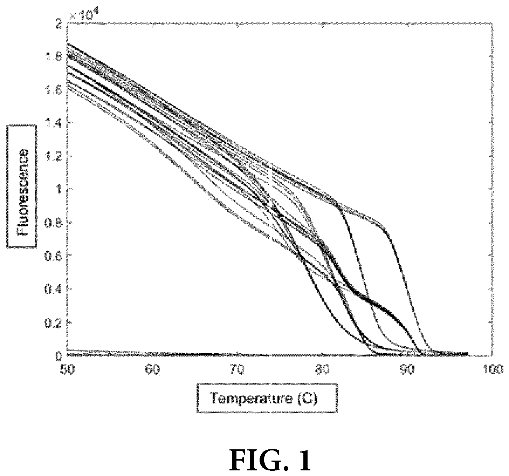

FIGS. 1-3 illustrate an example of automatic genotyping of melting curves in accordance with embodiments of the present disclosure. Specifically, FIG. 1 is a graphical representation of raw melting curves in accordance with the present disclosure. As can be seen in FIG. 1, the raw melting curve depicts the measured fluorescence over a range of temperatures during the melting step after PCR. In this example, the assay includes eight genotypes and a negative result.

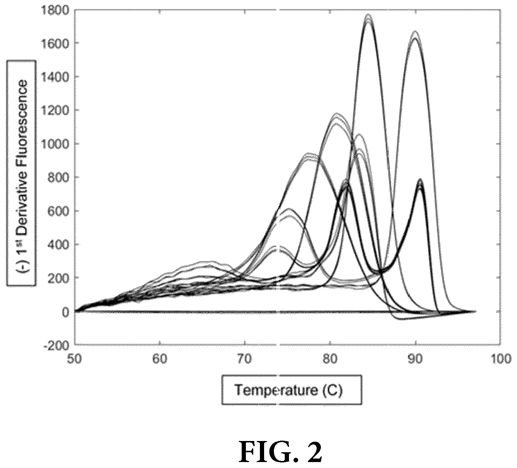

In accordance with embodiments of the present disclosure, the raw melting curves of FIG. 1 may be processed in order to accurately genotype the assay. Specifically, the raw melting curves of FIG. 1 may be processed by taking the negative derivative of each curve in FIG. 1. FIG. 2 is a graphical representation of processed raw melting curves of FIG. 1 resulting from taking the negative derivative of each curve shown in FIG. 1. As can be seen in FIG. 2, many of the resulting processed curves include Gaussian peaks, and there are groups of similar curves that are similar in the peak height, frequency, and location within the temperature range. While this can be seen visually, it would be burdensome and require extensive manual input to genotype these curves without knowing the number of actual genotypes in the assay, particularly because there happen to be a relatively large number of genotypes-in this case 8-in the assay.

Embodiments of the present disclosure provide methods to automatically genotype the processed melting curves without previous knowledge of the number of genotypes. Specifically, as will be described in further detail below, the processed melting curves may be used to automatically compile a series of difference matrices which in turn may be used to automatically cluster together curves of a single genotype.

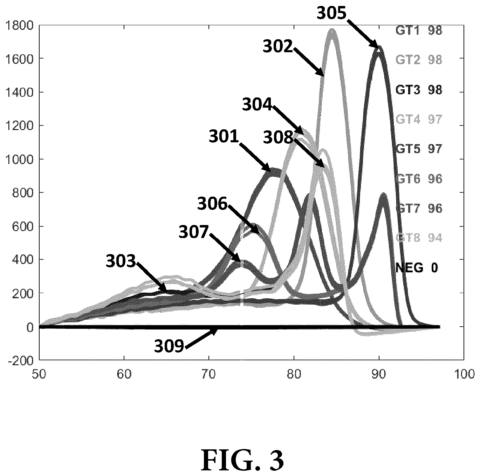

FIG. 3 is a graphical representation of processed raw melting curves of FIG. 2 after being genotyped in accordance with embodiments of the present disclosure. As can be seen from FIG. 3, each of the eight different genotypes (genotype 301 corresponding to GT1, genotype 302 corresponding to GT2, genotype 303 corresponding to GT3, and so on) have been identified along with a number of negative results (309 corresponding to NEG) for the assay.

In addition to identification of the genotypes, in some embodiments, a quality metric associated with a genotype can be determined. For example, a metric ranging from 0-100 can be determined, which is indicative of the "tightness" of the grouped curves in a given genotype. The closer the metric is to 100, the "tighter" a given cluster is. The quality metric determined for each of the genotypes identified in FIG. 3 is noted alongside each genotype and it can be seen that in this instance, the metric ranges from 94 to 98 for each of the eight identified genotypes, which represents a relatively "tight" fit within each group.

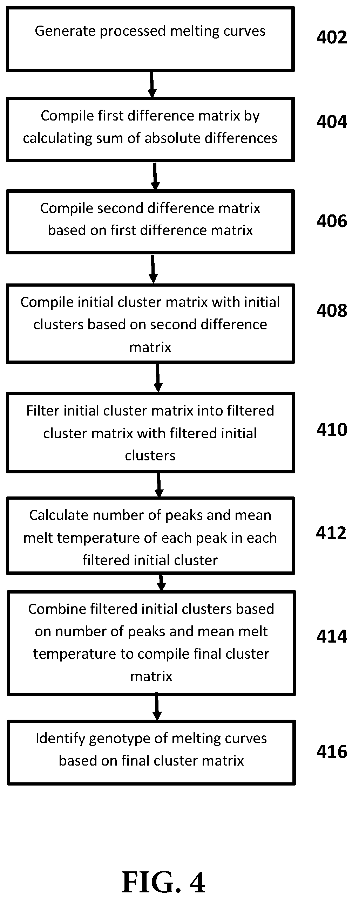

FIG. 4 shows a method 400 for automated genotyping of melting curves in accordance with embodiments of the present disclosure. At step 402, raw melting curves may be processed to generate processed melting curves. In some embodiments, the negative derivative of each of a plurality of raw melting curves may be calculated to generate a processed melting curve. The raw melting curves may be processed in any suitable way for calculating a negative derivative. While described herein as calculating a negative derivative, it will be understood that the raw melting curves may be processed in any suitable way that assists in ascertaining features of the curves (such as melting temperatures) that aid in genotyping the melting curves.

At step 404, a first difference matrix may be compiled based on the processed melting curves. In some embodiments, the first difference matrix may be compiled by taking the absolute difference in fluorescence value at each temperature between a given processed melting curve and each other processed melting curve. For example, the first difference matrix may have dimensions that include rows corresponding to each temperature unit at which a fluorescent value was measured over the range of temperatures during the post-PCR melting step and columns corresponding to the number of melting curves.

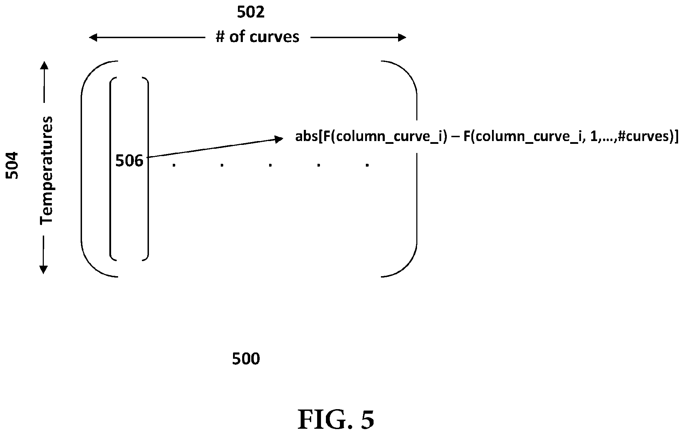

FIG. 5 is a graphical representation of the compilation of the first difference matrix 500 in accordance with embodiments of the present disclosure. As can be seen in FIG. 5, difference matrix may have a width 502 corresponding to the number curves in a given assay, and a height 504 corresponding to the discrete temperature units at which fluorescence measurements were taken. Each column 506 in matrix 500 may be calculated by determining the absolute difference between each curve and all other curves at each of the discrete temperatures.

At step 406, a second difference matrix may be compiled based on the contents of the first difference matrix compiled in step 404. In some embodiments, the second difference matrix may be compiled by determining, for each column of the first difference matrix, the sum of the row vector of the first difference matrix over all of the temperature units. In some embodiments, entries of the second difference matrix corresponding to a determination of a difference of a curve from itself are set to be Not a Number (NaN). The resulting second difference matrix provides a measure of similarity of each curve to each other curve, which will be used in subsequent steps to cluster the curves together in order to identify genotypes. In some embodiments, the second difference matrix may essentially be a vector with no temperature dimension, since it will contain, for each curve, summed values of the absolute distance from each of the other curves.

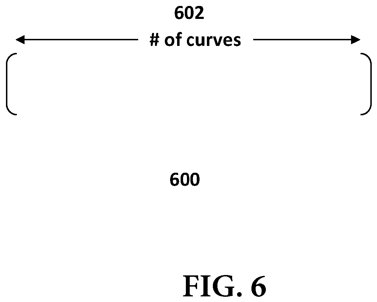

FIG. 6 is a graphical representation of the compilation of the second difference matrix in accordance with embodiments of the present disclosure. As can be seen, second difference matrix 600 does not have a temperature dimension, in contrast to first difference matrix 500 for example. Rather, difference matrix 600 has a width 602 that corresponds to the total number of curves in a given assay (similar to width 502 of first difference matrix 500). Difference matrix 600 is compiled by summing the row vectors of the first difference matrix over all of the temperature units, so that it contains, for each curve, summed values of the absolute distance from each of the other curves. Accordingly, difference matrix 600 may be a vector comprising the summed values of the absolute distance over all temperature units.

At step 408, an initial cluster matrix is compiled based on the second difference matrix compiled in step 406. The initial cluster matrix may be compiled by clustering together groups of the processed melting curves based on the second difference matrix. For example, an initial cluster matrix may be compiled by creating a matrix with dimensions including rows corresponding to the total number of melting curves and columns corresponding to one more than the total number of melting curves. The first column of the initial cluster matrix may contain a given curve number, and the second column of the initial cluster may contain a curve number corresponding to the minimum non-zero distance sum relative to that given curve number, which may be determined from the second difference matrix compiled in step 406. Subsequent columns of the initial cluster matrix may include additional curve numbers associated with the curve in the first column within a minimal distance.

In some embodiments, the initial cluster matrix may be compiled using clustering techniques. For example, the initial cluster matrix may be compiled using traditional k-means clustering. For example, k-means clustering may take as its input the temperature sum of the sum of absolute differences between a given curve and all other curves, which are reflected in the second difference matrix, and an input number of initial clusters. K-means clustering may then identify clusters by identifying the centroids (i.e. the center of mass) of clusters and identifying which curves belong to a given cluster based on the distance to the centroid. In some embodiments, the number of initial clusters may be a parameter that determines the maximum number of clusters to be identified by k-means clustering (or other clustering technique used). It will be understood that this parameter may be pre-determined, set by user input, or be dictated (automatically or by user input) by the circumstances of a given assay. In some embodiments, the number of initial clusters may be set to be a numberbetween 2 and 25. For instance, it may be preferable to set the initial number of clusters to be 15. In other embodiments, it may be preferable to expand the initial number of clusters to be 25. Using k-means clustering, the initial cluster matrix may be populated by identifying, in each row, a given curve number in the first column, followed by the curve numbers in the cluster whose centroid is a minimum distance from the curve in the first column, where the cluster is determined by k-means or other suitable clustering techniques.

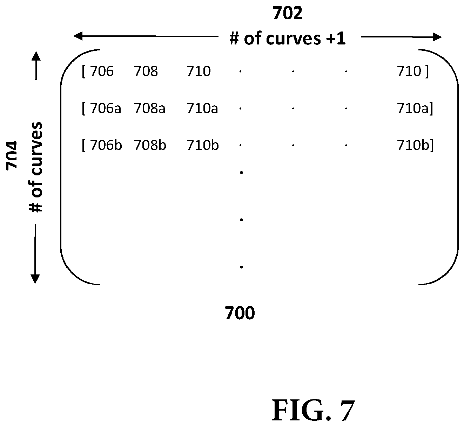

FIG. 7 is a graphical representation of the compilation of the cluster matrix in accordance with embodiments of the present disclosure. As can be seen in FIG. 7, the initial cluster matrix 700 may have width 702 corresponding to the number of curves + 1, and height 704 corresponding to the number of curves. The first column of each row may comprise a given curve number (706, 706a, 706b), the next column may comprise the curve number (708, 708a, 708b) with the minimum distance from the curve identified in the first column, and then subsequent columns (710, 710a, 710b) may include the remaining curve numbers within the cluster cluster whose centroid is a minimum distance from the curve in the first column. As described above, matrix 700 may be populated using k-means clustering, or other clustering techniques as described below.

Although k-means clustering is discussed above, other methods may be used to cluster the curves based on the difference matrices above. For example, any of heirarchical clustering, fuzzy C means clustering, mean shift clustering, density-based spatial clustering, and Gaussian mixture models may be used to cluster the curves using the first and second difference matrices described above.

At step 410, the initial cluster matrix compiled at step 408 is filtered to generate a filtered initial cluster matrix with filtered initial clusters. Filtering of the initial cluster matrix may include removing duplicate clusters, combining intersecting clusters, removing duplicate curves within clusters, designating negative clusters, and combining negative clusters if present, as will be described in further detail below.

In some embodiments, an initial filtering step may include removing duplicate clusters. In some embodiments, duplicate clusters may be removed by creating a new matrix with the non-zero elements of the initial cluster matrix. If two or more rows in the new matrix are exact duplicates (i.e. contain the exact same list of curves) then the duplicate rows are removed from the new matrix.

In some embodiments, a subsequent filtering step may include combining intersecting clusters so that a given curve is not present in more than one cluster. For example, combining intersecting clusters may include two passes through the cluster matrix. In the first pass, clusters which share a given curve are combined, but the original clusters are not removed to ensure that all intersecting clusters are evaluated. In the second pass, any remaining intersecting clusters are combined, but in this instance, the remaining clusters are removed. Since each potential cluster with intersecting curves is evaluated, this two-pass technique ensures that a single curve is not present in two different clusters.

In some embodiments, a subsequent filtering step includes removing duplicate curves within a cluster. For example, each cluster is evaluated to ensure that there are no duplicate curve numbers therein. If a duplicate curve number is found, it is removed from the cluster.

In some embodiments, a subsequent filtering step includes designating negative clusters. In some embodiments, clusters are designated negative based on a ratio of the maximum fluorescence of a cluster and the cluster with the highest maximum fluorescence. For example, the ratio of the maximum fluorescence of a cluster and the cluster with the highest maximum fluorescence may be compared to a cluster threshold. If the ratio is less than the cluster threshold, then that cluster is designated as a negative cluster. The cluster threshold may be determined experimentally to be a number that sets clusters of melt curves that are essentially flat to be designated as negative clusters. In some embodiments the cluster threshold may be 0.1 (or 10%). In some embodiments the cluster threshold may be 0.05 (or 5%). Once any negative clusters are designated, a subsequent step may be taken to combine multiple negative clusters. Additionally, a further step may be taken to check for the presence of a single negative curve within any remaining clusters. For example, the cluster with the lowest peak (once negative clusters have been designated) may be evaluated to determine if any of its curves are less than the cluster threshold. If any curves are less than the cluster threshold (i.e. if the ratio of the peak of said curve to the peak of the cluster with the highest maximum fluorescence is less than the cluster threshold) then those curves are set as negative and removed from the cluster.

At step 412, the mean melt temperature of each peak and the number of peaks in each remaining cluster is determined. At step 414, clusters containing the same number of peaks and a mean melt temperature within a threshold range may be combined. For example, clusters that have both (1) the same number of peaks, and (2) mean melt temperatures within +/- 1 degree C from each other may be combined. The resulting cluster matrix may be the final cluster matrix used for genotyping.

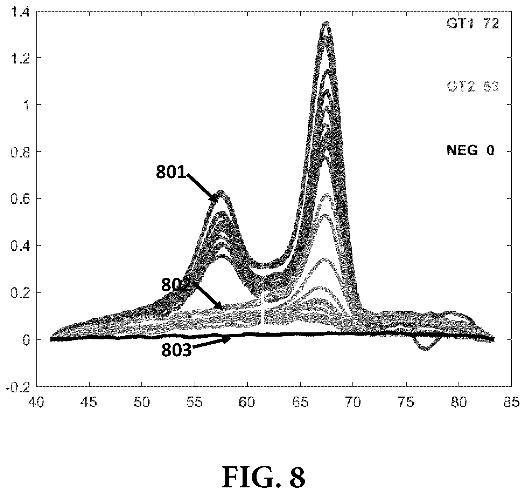

At step 416, genotypes of the melting curves are determined based on the final cluster matrix compiled at step 414. The genotyping may result in an output list of genotypes identified in a visual manner. For example, the curves may be visually displayed as in FIG. 3 (and/or FIGS. 8-12 which will be described below) with colors identifying like genotypes.

In some embodiments, a quality metric may be determined for each genotype group identified and also output for reference. Specifically, the quality metric of the group may be calculated using the equation:

$$qMetric = round\left\{100\left[1 - \frac{stdev(grp\_curves)}{mean(grp\_curves)}\right]\right\}$$

Where "grp_curves" represents a group of melting curves within a given cluster. The quality metric may be indicative of the tightness of a given cluster. For example, the closer the value of the quality metric is to 100, the tighter the curves in a given cluster is.

In some embodiments, a quality metric may be determined for each curve in a given genotype group. The quality metric of a given curve in a group may be calculated using the equation:

$$qMetric = 1 - 0.25\left[\frac{abs(mean(f - median(f)))}{mean(stdev(f))}\right]$$

Where f designates a given processed melting curve, and the quality metric is indicative of how close a curve is to the median of the cluster associated with the genotype.

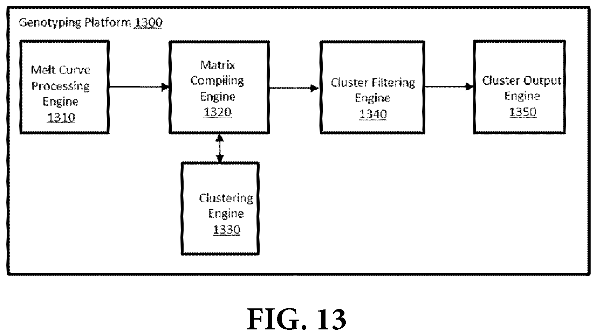

FIG. 13 depicts a diagram illustrating an example of a genotyping platform 1300, in accordance with implementations of the current disclosure. Genotyping platform 1300 may include various engines that, cooperatively, cluster and genotype melting curves of an assay as described above with reference to FIGS. 1-4.

Genotyping platform 1300 may include melt curve processing engine 1310, matrix compiling engine 1320, clustering engine 1330, cluster filtering engine 1340, and cluster output engine 1350. In some implementations, one or more aspects, features, and/or operations of the engines 1310, 1320, 1330, 1340, and 1350 may be combined in various combinations.

Melt curve processing engine 1310 may receive as an input raw melting curves to be genotyped as described herein. Melting curve processing engine 1310 may be configured to process the raw melting curves as described above with reference to step 402 of method 400. For example, melting curve processing engine 1310 may be configured to calculate the negative first derivative of the raw melting curves.

Matrix compiling engine 1320 may receive, from melting curve processing engine 1310, processed raw melting curves to be genotyped. Matrix compiling engine 1320 may be configured to compile difference matrices as described above with reference to step 404 and 406 of method 400. For example, matrix compiling engine 1320 may be configured to compile first difference matrix 500 as described in FIG. 5 based on the processed raw melting curves and then compile second difference matrix 600 as described in FIG. 6 based on the first difference matrix.

Clustering engine 1330 may receive the difference matrices from matrix compiling engine and use them to cluster the processed raw melting curves as described above. For example, clustering engine 1330 may be configured to use k-means clustering to identify clusters based on the second difference matrix. Clustering engine 1330 may alternatively use other clustering techniques as described above, including hierarchical clustering, fuzzy C means clustering, mean shift clustering, density-based spatial clustering, and Gaussian mixture models to cluster the curves using the first and second difference matrices described above.

Matrix compiling engine 1320 may receive the set of clusters identified by clustering engine 1330 and compile an initial cluster matrix as described above in step 408 of method 400. For example, matrix compiling engine 1320 may be configured to compile an initial cluster matrix such as matrix 700 described in FIG. 7.

Cluster filtering engine 1340 may receive the initial cluster matrix from matrix compiling engine 1320. Cluster filtering engine 1340 may be configured to filter the initial cluster matrix as described in steps 410-414 of method 400. For example, cluster filtering engine 1340 may be configured to remove duplicate clusters, combine intersecting clusters, remove duplicate curves within clusters, designate negative clusters, and combine negative clusters if present from the initial cluster matrix as described above to result in a filtered cluster matrix. Cluster filtering engine 1340 may also be configured to combine clusters based on an evaluation of the number of peaks and melt temperature and compile the final cluster matrix as described above with reference to step 414 of method 400.

Cluster output engine 1350 may receive the final cluster matrix from cluster filtering engine 1340. Cluster output engine 1350 may be configured to identify the genotype of the melting curves based on the final cluster matrix and output the results. Cluster output engine 1350 may also be configured to calculate the quality metrics for each genotype and/or each curve as described above. For example, cluster output engine 1350 may output graphical representations (e.g. such as those shown in FIGS 3 and FIGS. 8-12) showing genotypes of each of the curves in a given assay under consideration.

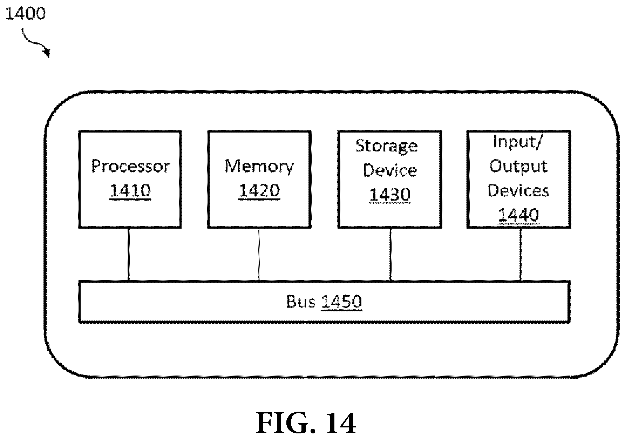

FIG. 14 depicts a block diagram illustrating an example of computing system 1400, in accordance with some example embodiments. Referring to FIGs. 1-4 and 13-14, the computing system 1400 may be used to implement the genotyping platform 1300, method 400 and/or any components therein.

As shown in FIG. 14, computing system 1400 can include a processor 1410, a memory 1420, a storage device 1430, and input/output devices 1440. Processor 1410, memory 1420, storage device 1430, and input/output devices 1440 can be interconnected via system bus 1450. Processor 1410 is capable of processing instructions for execution within the computing system 1400. Such executed instructions can implement one or more components of, for example, genotyping platform 100, method 400 and/or any components therein. In some example embodiments, processor 1410 can be a single-threaded processor. Alternately, processor 1410 can be a multi-threaded processor. Processor 1410 is capable of processing instructions stored in memory 1420 and/or on the storage device 1430 to display graphical information for a user interface provided via the input/output device 1440.

Memory 1420 is a computer readable medium such as volatile or non-volatile that stores information within computing system 1400. Memory 1420 can store data structures representing configuration object databases, for example. Storage device 1430 is capable of providing persistent storage for computing system 1400. Storage device 1430 can be a floppy disk device, a hard disk device, an optical disk device, or a tape device, or other suitable persistent storage means. Input/output device 1440 provides input/output operations for the computing system 1400. In some example embodiments, input/output device 1440 includes a keyboard and/or pointing device. In various implementations, the input/output device 1440 includes a display unit for displaying graphical user interfaces.

According to some example embodiments, input/output device 1440 can provide input/output operations for a network device. For example, input/output device 1440 can include Ethernet ports or other networking ports to communicate with one or more wired and/or wireless networks (e.g., a local area network (LAN), a wide area network (WAN), the Internet).

In some example embodiments, computing system 1400 can be used to execute various interactive computer software applications that can be used for organization, analysis and/or storage of data in various formats. Alternatively, computing system 1400 can be used to execute any type of software applications. These applications can be used to perform various functionalities, e.g., planning functionalities (e.g., generating, managing, editing of spreadsheet documents, word processing documents, and/or any other objects, etc.), computing functionalities, communications functionalities, etc. The applications can include various add-in functionalities or can be standalone computing products and/or functionalities. Upon activation within the applications, the functionalities can be used to generate the user interface provided via input/output device 1440. The user interface can be generated and presented to a user by computing system 1400 (e.g., on a computer screen monitor, etc.).

One or more aspects or features of the subject matter described herein can be realized in digital electronic circuitry, integrated circuitry, specially designed application-specific integrated circuits (ASICs), field programmable gate arrays (FPGAs) computer hardware, firmware, software, and/or combinations thereof. These various aspects or features can include implementation in one or more computer programs that are executable and/or interpretable on a programmable system including at least one programmable processor, which can be special or general purpose, coupled to receive data and instructions from, and to transmit data and instructions to, a storage system, at least one input device, and at least one output device. The programmable system or computing system may include clients and servers. A client and server are generally remote from each other and typically interact through a communication network. The relationship of client and server arises by virtue of computer programs running on the respective computers and having a client-server relationship to each other.

These computer programs, which can also be referred to as programs, software, software applications, applications, components, or code, include machine instructions for a programmable processor, and can be implemented in a high-level procedural and/or object-oriented programming language, and/or in assembly/machine language. As used herein, the term "machine-readable medium" refers to any computer program product, apparatus and/or device, such as for example magnetic discs, optical disks, memory, and Programmable Logic Devices (PLDs), used to provide machine instructions and/or data to a programmable processor, including a machine-readable medium that receives machine instructions as a machine-readable signal. The term "machine-readable signal" refers to any signal used to provide machine instructions and/or data to a programmable processor. The machine-readable medium can store such machine instructions non-transitorily, such as for example as would a non-transient solid-state memory or a magnetic hard drive or any equivalent storage medium. The machine-readable medium can alternatively or additionally store such machine instructions in a transient manner, such as for example, as would a processor cache or other random access memory associated with one or more physical processor cores.

To provide for interaction with a user, one or more aspects or features of the subject matter described herein can be implemented on a computer having a display device, such as for example a cathode ray tube (CRT) or a liquid crystal display (LCD) or a light emitting diode (LED) monitor for displaying information to the user and a keyboard and a pointing device, such as for example a mouse or a trackball, by which the user may provide input to the computer. Other kinds of devices can be used to provide for interaction with a user as well. For example, feedback provided to the user can be any form of sensory feedback, such as for example visual feedback, auditory feedback, or tactile feedback; and input from the user may be received in any form, including acoustic, speech, or tactile input. Other possible input devices include touch screens or other touch-sensitive devices such as single or multi-point resistive or capacitive track pads, voice recognition hardware and software, optical scanners, optical pointers, digital image capture devices and associated interpretation software, and the like.

Embodiments of the present invention will be further described in the following examples, which do not limit the scope of the invention described in the claims.

## EXAMPLES

### Example 1

FIG. 8 is a graphical representation of genotyped processed raw melting curves in accordance with embodiments of the present disclosure. In particular, the assay genotyped was a BCR-ABL mutation assay, often used to diagnose or rule out chronic myeloid leukemia or a form of acute lymphoblastic leukemia (ALL) called Ph-positive ALL. As can be seen in FIG. 8, two genotypes 801 (GT1) and 802 (GT2) were identified, with quality metrics of 72 and 53 respectively.

### Example 2

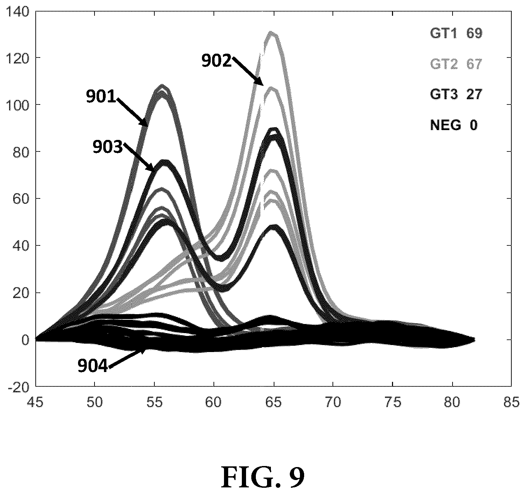

FIG. 9 is a graphical representation of genotyped processed raw melting curves in accordance with embodiments of the present disclosure. In particular, the assay genotyped was for Apolipoprotein (apo) E. The Apo E alleles may be used to identify risk of coronary heart disease and Alzheimer disease, among others. As can be seen in FIG. 9, three genotypes 901 (GT1), 902 (GT2), and 903 (GT3) were identified, with quality metrics of 69, 67, and 27 respectively.

### Example 3

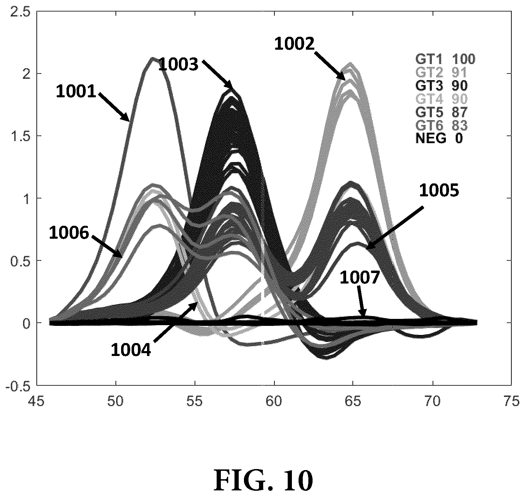

FIG. 10 is a graphical representation of genotyped processed raw melting curves in accordance with embodiments of the present disclosure. In particular, the assay genotyped was for the hemochromatosis gene (HFE). As can be seen in FIG. 10, six genotypes 1001 (GT1), 1002 (GT2), 1003 (GT3), 1004 (GT4), 1005 (GT5), and 1006 (GT6) were identified, with quality metrics of 100, 91, 90, 90, 87, and 83 respectively.

### Example 4

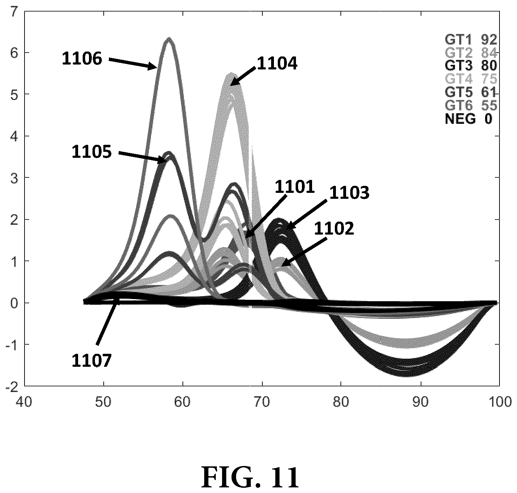

FIG. 11 is a graphical representation of genotyped processed raw melting curves in accordance with embodiments of the present disclosure. In particular, the assay was for factor V Leiden (FV), prothrombin (FII), and methylenetetrahydrofolate reductase (MTHFR) genes which are commonly investigated inherited risk factors for developing venous thromboembolism (VTE). As can be seen in FIG. 11, six genotypes 1101 (GT1), 1102 (GT2), 1103 (GT3), 1104 (GT4), 1105 (GT5), and 1106 (GT6) were identified, with quality metrics of 92, 84, 80, 75, 61, and 55 respectively.

### Example 5

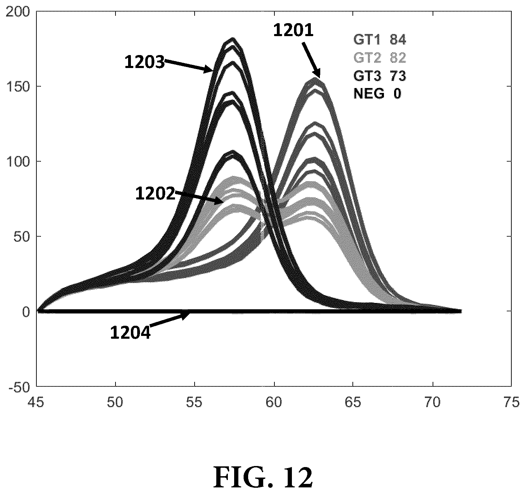

FIG. 12 is a graphical representation of genotyped processed raw melting curves in accordance with embodiments of the present disclosure. In particular, the assay was for lactose intolerance-related targets. As can be seen in FIG. 12, three genotypes 1201 (GT1), 1202 (GT2), and 1203 (GT3) were identified, with quality metrics of 84, 82, and 73 respectively.

In the descriptions above and in the claims, phrases such as "at least one of" or "one or more of" may occur followed by a conjunctive list of elements or features. The term "and/or" may also occur in a list of two or more elements or features. Unless otherwise implicitly or explicitly contradicted by the context in which it used, such a phrase is intended to mean any of the listed elements or features individually or any of the recited elements or features in combination with any of the other recited elements or features. For example, the phrases "at least one of A and B;" "one or more of A and B;" and "A and/or B" are each intended to mean "A alone, B alone, or A and B together." A similar interpretation is also intended for lists including three or more items. For example, the phrases "at least one of A, B, and C;" "one or more of A, B, and C;" and "A, B, and/or C" are each intended to mean "A alone, B alone, C alone, A and B together, A and C together, B and C together, or A and B and C together." Use of the term "based on," above and in the claims is intended to mean, "based at least in part on," such that an unrecited feature or element is also permissible.

The subject matter described herein can be embodied in systems, apparatus, methods, and/or articles depending on the desired configuration. The implementations set forth in the foregoing description do not represent all implementations consistent with the subject matter described herein. Instead, they are merely some examples consistent with aspects related to the described subject matter. Although a few variations have been described in detail above, other modifications or additions are possible. In particular, further features and/or variations can be provided in addition to those set forth herein. For example, the implementations described above can be directed to various combinations and subcombinations of the disclosed features and/or combinations and subcombinations of several further features disclosed above. In addition, the logic flows depicted in the accompanying figures and/or described herein do not necessarily require the particular order shown, or sequential order, to achieve desirable results. Other implementations may be within the scope of the following claims.

**Claims**

1.  An automated method for genotyping a plurality of melting curves comprising fluorescent measurements over a range of temperatures, the method comprising:

    generating a plurality of processed melting curves by calculating the negative first derivative of each of the plurality of melting curves over the range of temperatures;

    compiling a first difference matrix based on the processed melting curves by calculating, for each of the processed melting curves, a sum of absolute differences between a given processed melting curve and each of the other processed melting curves over the range of temperatures;

    compiling a second difference matrix based on the first difference matrix by summing, for each column of the first difference matrix, each row vector over the range of temperatures;

    compiling an initial cluster matrix based on the second difference matrix, wherein each row in the initial cluster matrix identifies an initial cluster in the set of initial clusters;

    filtering the initial cluster matrix into a filtered initial cluster matrix comprising a set of filtered initial clusters, wherein each of the processed melting curves is assigned to a single filtered initial cluster;

    calculating a number of peaks and a mean melt temperature of each peak in each filtered initial cluster of the filtered initial cluster matrix;

    combining filtered initial clusters with the same number of peaks and a mean melt temperature within a specified temperature threshold to compile a final cluster matrix; and

    identifying a genotype of each of the plurality of melting curves based on the final cluster matrix.

2.  The method of claim 1, wherein the initial cluster matrix is compiled by performing k-means clustering.

3.  The method of claim 2, wherein each row of the initial cluster matrix comprises:

    a first column identifying a first processed melting curve;

    a second column identifying a second processed melting curve with a minimum non-zero distance from the first processed melting curve of the first column, the minimum non-zero distance determined based on the second difference matrix; and

    subsequent columns identifying additional processed melting curves within a cluster with the first processed melting curve of the first column, wherein the cluster is identified based on the distance between a centroid of the cluster and the first processed melting curve.

4.  The method of claim 2, wherein the k-means clustering is performed based on an input number of initial clusters.

5.  The method of claim 1, wherein filtering the initial cluster matrix comprises removing duplicate rows from the initial cluster matrix.

6.  The method of claim 5, wherein filtering the initial cluster matrix further comprises combining intersecting clusters that contain common processed melting curves.

7.  The method of claim 6, wherein combining intersecting clusters comprises the steps of:

    (a) identifying, in a first pass, initial clusters in the set of initial clusters that contain a common processed melting curve;

    (b) combining each of the clusters identified in step (a);

    (c) identifying, in a second pass, any remaining clusters that contain common processed melting curves and combining said remaining clusters; and

    (d) removing the initial clusters containing common processed melting curves such that none of the processed melting curves are present in more than one filtered initial cluster.

8. The method of claim 5, wherein filtering the initial cluster matrix further comprises identifying clusters with duplicate processed melting curves, and removing said duplicates.

9. The method of claim 5, wherein filtering the initial cluster matrix further comprises the steps of:

(i) identifying a negative cluster based on a comparison of a maximum signal of the cluster to a cluster threshold;
(ii) merging any negative clusters identified in step (i).

10. The method of claim 9, wherein identifying a negative cluster comprises:

(aa) determining a ratio of a maximum fluorescence value in a given cluster and a maximum fluorescence in a cluster with the highest fluorescence; and
(bb) comparing the ratio determined at step (aa) to the cluster threshold.

11. The method of claim 10, wherein the cluster threshold is 0.1.

12. The method of claim 9, wherein filtering the initial cluster matrix further comprises:

(iii) identifying a cluster comprising a peak that is less than the peaks of each of the other clusters;
(iv) comparing processed melting curves in the cluster identified in step (iii) to the cluster threshold;
(v) setting any of the processed melting curves that are less than the cluster threshold as negative processed melting curves and removing said negative processed melting curves from the cluster.

13. The method of claim 1, wherein the specified temperature threshold is 1 ° C.

14. The method of claim 1, further comprising determining a quality metric associated with each of the identified genotypes according to the formula: $qMetric = round\left\{100\left[1 - \dfrac{stdev(grp\_curves)}{mean(grp\_curves)}\right]\right\}$ , wherein "grp_curves" comprises a group of melting curves within a given cluster.

15. The method of claim 1, further comprising determining a quality metric associated with each processed melting curve within a cluster according to the formula: $qMetric = 1 - 0.25\left[\dfrac{abs(mean(f - median(f)))}{mean(stdev(f))}\right]$ , wherein "f" designates a given processed melting curve.

16. The method of claim 1, wherein the initial cluster matrix is compiled by performing at least one of hierarchical clustering, fuzzy C means clustering, mean shift clustering, density-based spatial clustering, and Gaussian mixture models.

**Patentansprüche**

1. Automatisiertes Verfahren zum Genotypisieren einer Vielzahl von Schmelzkurven, die Fluoreszenzmessungen über einen Temperaturbereich umfassen, wobei das Verfahren Folgendes umfasst:

Erzeugen einer Vielzahl von verarbeiteten Schmelzkurven durch Berechnen der negativen ersten Ableitung jeder der Vielzahl von Schmelzkurven über den Temperaturbereich;
Erstellen einer ersten Differenzmatrix basierend auf den verarbeiteten Schmelzkurven durch Berechnen, für jede der verarbeiteten Schmelzkurven, einer Summe von absoluten Differenzen zwischen einer gegebenen verarbeiteten Schmelzkurve und jeder der anderen verarbeiteten Schmelzkurven über den Temperaturbereich;
Erstellen einer zweiten Differenzmatrix basierend auf der ersten Differenzmatrix durch Summieren, für jede Spalte der ersten Differenzmatrix, jedes Zeilenvektors über den Temperaturbereich;

Erstellen einer Initialclustermatrix basierend auf der zweiten Differenzmatrix, wobei jede Zeile in der Initialclustermatrix einen Initialcluster in dem Satz von Initialclustern identifiziert;

Filtern der Initialclustermatrix in eine gefilterte Initialclustermatrix, umfassend einen Satz von gefilterten Initialclustern, wobei jede der verarbeiteten Schmelzkurven einem einzelnen gefilterten Initialcluster zugewiesen ist;

Berechnen einer Anzahl von Peaks und einer mittleren Schmelztemperatur von jedem Peak in jedem gefilterten Initialcluster der gefilterten Initialclustermatrix;

Kombinieren von gefilterten Initialclustern mit der gleichen Anzahl von Peaks und einer mittleren Schmelztemperatur innerhalb eines spezifizierten Temperaturschwellenwerts, um eine Finalclustermatrix zu erstellen; und

Identifizieren eines Genotyps von jeder der Vielzahl von Schmelzkurven basierend auf der Finalclustermatrix.

2. Verfahren nach Anspruch 1, wobei die Initialclustermatrix durch Durchführen von k-Means-Clustern erstellt wird.

3. Verfahren nach Anspruch 2, wobei jede Zeile der Initialclustermatrix Folgendes umfasst:

eine erste Spalte, die eine erste verarbeitete Schmelzkurve identifiziert;

eine zweite Spalte, die eine zweite verarbeitete Schmelzkurve mit einem minimalen Nicht-Null-Abstand von der ersten verarbeiteten Schmelzkurve der ersten Spalte identifiziert, wobei der minimale Nicht-Null-Abstand basierend auf der zweiten Differenzmatrix bestimmt wird; und

nachfolgende Spalten, die zusätzliche verarbeitete Schmelzkurven innerhalb eines Clusters mit der ersten verarbeiteten Schmelzkurve der ersten Spalte identifizieren, wobei der Cluster basierend auf dem Abstand zwischen einem Schwerpunkt des Clusters und der ersten verarbeiteten Schmelzkurve identifiziert wird.

4. Verfahren nach Anspruch 2, wobei das k-Means-Clustern basierend auf einer Eingabeanzahl von Initialclustern durchgeführt wird.

5. Verfahren nach Anspruch 1, wobei das Filtern der Initialclustermatrix das Entfernen von doppelten Zeilen aus der Initialclustermatrix umfasst.

6. Verfahren nach Anspruch 5, wobei das Filtern der Initialclustermatrix ferner das Kombinieren von sich schneidenden Clustern umfasst, die gemeinsame verarbeitete Schmelzkurven enthalten.

7. Verfahren nach Anspruch 6, wobei das Kombinieren von sich schneidenden Clustern die folgenden Schritte umfasst:

(a) Identifizieren, in einem ersten Durchgang, von Initialclustern in dem Satz von Initialclustern, die eine gemeinsame verarbeitete Schmelzkurve enthalten;

(b) Kombinieren von jedem der in Schritt (a) identifizierten Cluster;

(c) Identifizieren, in einem zweiten Durchgang, von verbleibenden Clustern, die gemeinsame verarbeitete Schmelzkurven enthalten, und Kombinieren der verbleibenden Cluster; und

(d) Entfernen der Initialcluster, die gemeinsame verarbeitete Schmelzkurven enthalten, sodass keine der verarbeiteten Schmelzkurven in mehr als einem gefilterten Initialcluster vorhanden sind.

8. Verfahren nach Anspruch 5, wobei das Filtern der Initialclustermatrix ferner das Identifizieren von Clustern mit doppelten Schmelzkurven und das Entfernen der Duplikate umfasst.

9. Verfahren nach Anspruch 5, wobei das Filtern der Initialclustermatrix ferner die folgenden Schritte umfasst:

(i) Identifizieren eines negativen Clusters basierend auf einem Vergleich eines maximalen Signals des Clusters mit einem Clusterschwellenwert;

(ii) Zusammenführen aller in Schritt (i) identifizierten negativen Clustern.

10. Verfahren nach Anspruch 9, wobei das Identifizieren eines negativen Clusters Folgendes umfasst:

(aa) Bestimmen eines Verhältnisses eines maximalen Fluoreszenzwerts in einem gegebenen Cluster und einer maximalen Fluoreszenz in einem Cluster mit der höchsten Fluoreszenz; und

(bb) Vergleichen des in Schritt (aa) bestimmten Verhältnisses mit dem Clusterschwellenwert.

**11.** Verfahren nach Anspruch 10, wobei der Clusterschwellenwert 0,1 beträgt.

**12.** Verfahren nach Anspruch 9, wobei das Filtern der Initialclustermatrix ferner Folgendes umfasst:

(iii) Identifizieren eines Clusters, der einen Peak umfasst, der geringer als die Peaks von jedem der anderen Cluster ist;
(iv) Vergleichen von verarbeiteten Schmelzkurven in dem in Schritt (iii) identifizierten Cluster mit dem Clusterschwellenwert;
(v) Festlegen von beliebigen der verarbeiteten Schmelzkurven, die geringer als der Clusterschwellenwert sind, als negative verarbeitete Schmelzkurven und Entfernen der negativen verarbeiteten Schmelzkurven aus dem Cluster.

**13.** Verfahren nach Anspruch 1, wobei der spezifizierte Temperaturschwellenwert 1°C beträgt.

**14.** Verfahren nach Anspruch 1, ferner umfassend das Bestimmen einer Qualitätsmetrik, die mit jedem der identifizierten Genotypen assoziiert ist, gemäß der Formel: $qMetric = round \left\{ 100 \left[ 1 - \frac{stdev \ (grp \_ curves)}{mean \ (grp \_ curves)} \right] \right\}$ , wobei "grp_curves" eine Gruppe von Schmelzkurven in einem gegebenen Cluster umfasst.

**15.** Verfahren nach Anspruch 1, ferner umfassend das Bestimmen einer Qualitätsmetrik, die mit jeder verarbeiteten Schmelzkurve innerhalb eines Clusters assoziiert ist, gemäß der Formel: $qMetric = 1 - 0.25 \left[ \frac{abs \ (mean \ (f - median \ (f)))}{mean \ (stdev(f))} \right]$ , wobei "f" eine gegebene verarbeitete Schmelzkurve bezeichnet.

**16.** Verfahren nach Anspruch 1, wobei die Initialclustermatrix durch Durchführen von mindestens einem der Folgenden erstellt wird: hierarchisches Clustern, Fuzzy-C-Means-Clustern, Mean-Shift-Clustern, dichtebasiertes räumliches Clustern und Gauß'sche Mischmodelle.

**Revendications**

**1.** Procédé automatisé pour génotyper une pluralité de courbes de fusion comprenant des mesures de fluorescence sur une plage de températures, le procédé comprenant :

la génération d'une pluralité de courbes de fusion traitées en calculant la dérivée première négative de chacune de la pluralité de courbes de fusion sur la plage de températures ;
la compilation d'une première matrice de différences en se basant sur les courbes de fusion traitées en calculant, pour chacune des courbes de fusion traitées, une somme de différences absolues entre une courbe de fusion traitée donnée et chacune des autres courbes de fusion traitées sur la plage de températures ;
la compilation d'une deuxième matrice de différences en se basant sur la première matrice de différences en additionnant, pour chaque colonne de la première matrice de différences, chaque vecteur de ligne sur la plage de températures ;
la compilation d'une matrice de regroupements initiaux en se basant sur la deuxième matrice de différences, dans lequel chaque ligne dans la matrice de regroupements initiaux identifie un regroupement initial dans l'ensemble de regroupements initiaux ;
le filtrage de la matrice de regroupements initiaux en une matrice de regroupements initiaux filtrés comprenant un ensemble de regroupements initiaux filtrés, dans lequel chacune des courbes de fusion traitées est attribuée à un unique regroupement initial filtré ;
le calcul d'un nombre de pics et d'une température de fusion moyenne de chaque pic dans chaque regroupement initial filtré de la matrice de regroupements initiaux filtrés ;
la combinaison des regroupements initiaux filtrés avec le même nombre de pics et une température de fusion moyenne au sein d'un seuil de température spécifié pour compiler une matrice de regroupements finaux ; et
l'identification d'un génotype de chacune de la pluralité de courbes de fusion en se basant sur la matrice de regroupements finaux.

**2.** Procédé selon la revendication 1, dans lequel la matrice de regroupements initiaux est compilée en réalisant un regroupement par k-moyennes.

**3.** Procédé selon la revendication 2, dans lequel chaque ligne de la matrice de regroupements initiaux comprend :

une première colonne identifiant une première courbe de fusion traitée ;
une deuxième colonne identifiant une deuxième courbe de fusion traitée avec une distance non nulle minimale depuis la première courbe de fusion traitée de la première colonne, la distance non nulle minimale étant déterminée en se basant sur la deuxième matrice de différences ; et
des colonnes suivantes identifiant des courbes de fusion traitées supplémentaires au sein d'un regroupement avec la première courbe de fusion traitée de la première colonne, dans lequel le regroupement est identifié en se basant sur la distance entre un centroïde du regroupement et la première courbe de fusion traitée.

**4.** Procédé selon la revendication 2, dans lequel le regroupement par k-moyennes est réalisé en se basant sur un nombre d'entrées de regroupements initiaux.

**5.** Procédé selon la revendication 1, dans lequel le filtrage de la matrice de regroupements initiaux comprend la suppression des doublons de lignes de la matrice de regroupements initiaux.

**6.** Procédé selon la revendication 5, dans lequel le filtrage de la matrice de regroupements initiaux comprend en outre la combinaison de regroupements se chevauchant qui contiennent des courbes de fusion traitées communes.

**7.** Procédé selon la revendication 6, dans lequel la combinaison de regroupements se chevauchant comprend les étapes de :

(a) identification, lors d'un premier passage, de regroupements initiaux dans l'ensemble de regroupements initiaux qui contiennent une courbe de fusion traitée commune ;
(b) combinaison de chacun des regroupements identifiés à l'étape (a) ;
(c) identification, lors d'un deuxième passage, de tous les regroupements restants qui contiennent des courbes de fusion traitées communes et combinaison desdits regroupements restants ; et
(d) suppression des regroupements initiaux contenant des courbes de fusion traitées communes de telle sorte qu'aucune des courbes de fusion traitées n'est présente dans plus d'un regroupement initial filtré.

**8.** Procédé selon la revendication 5, dans lequel le filtrage de la matrice de regroupements initiaux comprend en outre l'identification de regroupements avec des doublons de courbes de fusion traitées, et la suppression desdits doublons.

**9.** Procédé selon la revendication 5, dans lequel le filtrage de la matrice de regroupements initiaux comprend en outre les étapes de :

(i) identification d'un regroupement négatif en se basant sur une comparaison d'un signal maximal du regroupement avec un seuil de regroupement ;
(ii) fusion de tous les regroupements négatifs identifiés à l'étape (i).

**10.** Procédé selon la revendication 9, dans lequel l'identification d'un regroupement négatif comprend :

(aa) la détermination d'un rapport d'une valeur de fluorescence maximale dans un regroupement donné et d'une fluorescence maximale dans un regroupement avec la fluorescence la plus élevée ; et
(bb) la comparaison du rapport déterminé à l'étape (aa) avec le seuil de regroupement.

**11.** Procédé selon la revendication 10, dans lequel le seuil de regroupement est de 0,1.

**12.** Procédé selon la revendication 9, dans lequel le filtrage de la matrice de regroupements initiaux comprend en outre :

(iii) l'identification d'un regroupement comprenant un pic qui est inférieur aux pics de chacun des autres regroupements ;
(iv) la comparaison des courbes de fusion traitées dans le regroupement identifié à l'étape (iii) avec le seuil de regroupement ;

(v) la définition de toutes les courbes de fusion traitées qui sont inférieures au seuil de regroupement comme courbes de fusion traitées négatives et la suppression desdites courbes de fusion traitées négatives du regroupement.

**13.** Procédé selon la revendication 1, dans lequel le seuil de température spécifié est de 1 °C.

**14.** Procédé selon la revendication 1, comprenant en outre la détermination d'une métrique de qualité associée à chacun des génotypes identifiés conformément à la formule : $Métrique_q = arrondi\left\{100\left[1 - \frac{ET(grp\_courbes)}{moy(grp\_courbes)}\right]\right\}$, dans lequel « grp_courbes » comprend un groupe de courbes de fusion au sein d'un regroupement donné.

**15.** Procédé selon la revendication 1, comprenant en outre la détermination d'une métrique de qualité associée à chaque courbe de fusion traitée au sein d'un regroupement conformément à la formule :

$$Métrique_q = 1 - 0{,}25\left[\frac{abs(moy(f - médiane(f)))}{moy(ET(f))}\right],$$

dans lequel « f » désigne une courbe de fusion traitée donnée.

**16.** Procédé selon la revendication 1, dans lequel la matrice de regroupements initiaux est compilée en réalisant au moins un parmi un regroupement hiérarchique, un regroupement flou par C-moyennes, un regroupement par décalage moyen, un regroupement spatial basé sur la densité et des modèles de mélange gaussien.

FIG. 1

**FIG. 2**

FIG. 3

```
┌─────────────────────────────────┐
│   Generate processed melting curves   │   402
└─────────────────────────────────┘
              │
              ▼
┌─────────────────────────────────┐
│   Compile first difference matrix by   │   404
│   calculating sum of absolute differences │
└─────────────────────────────────┘
              │
              ▼
┌─────────────────────────────────┐
│   Compile second difference matrix    │   406
│   based on first difference matrix     │
└─────────────────────────────────┘
              │
              ▼
┌─────────────────────────────────┐
│   Compile initial cluster matrix with initial │   408
│   clusters based on second difference  │
│   matrix                              │
└─────────────────────────────────┘
              │
              ▼
┌─────────────────────────────────┐
│   Filter initial cluster matrix into filtered │   410
│   cluster matrix with filtered initial │
│   clusters                            │
└─────────────────────────────────┘
              │
              ▼
┌─────────────────────────────────┐
│   Calculate number of peaks and mean  │   412
│   melt temperature of each peak in each │
│   filtered initial cluster            │
└─────────────────────────────────┘
              │
              ▼
┌─────────────────────────────────┐
│   Combine filtered initial clusters based │   414
│   on number of peaks and mean melt    │
│   temperature to compile final cluster │
│   matrix                              │
└─────────────────────────────────┘
              │
              ▼
┌─────────────────────────────────┐
│   Identify genotype of melting curves  │   416
│   based on final cluster matrix        │
└─────────────────────────────────┘
```

# FIG. 4

**502**

**# of curves**

**504**

**Temperatures**

**506**

$abs[F(column\_curve\_i) - F(column\_curve\_i, 1, ..., \#curves)]$

**500**

**FIG. 5**

602
# of curves

600

FIG. 6

**702**

← # of curves +1 →

**704**
**# of curves**

[ 706   708   710   ·   ·   ·   710 ]

[ 706a  708a  710a  ·   ·   ·   710a]

[ 706b  708b  710b  ·   ·   ·   710b]

·

·

·

**700**

# FIG. 7

FIG. 8

FIG. 9

**FIG. 10**

FIG. 11

**FIG. 12**

**FIG. 13**

1400

FIG. 14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007035806 A2